(19) Europäisches Patentamt
European Patent Office
Office européen des brevets

(11) Veröffentlichungsnummer: **0 039 405**
**A2**

(12) **EUROPÄISCHE PATENTANMELDUNG**

(21) Anmeldenummer: **81102090.8**

(22) Anmeldetag: **20.03.81**

(51) Int. Cl.³: **C 07 D 249/08**, A 01 N 43/64 // C07C49/255

(30) Priorität: **02.04.80 DE 3012824**

(43) Veröffentlichungstag der Anmeldung: **11.11.81 Patentblatt 81/45**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Kraatz, Udo, Dr., Körnerstrasse 6, D-5090 Leverkusen 1 (DE)**
Erfinder: **Jäger, Gerhard, Dr., Gellertstrasse 18, D-5090 Leverkusen 1 (DE)**
Erfinder: **Büchel, Karl Heinz, Prof. Dr., Haus an der Dachsdelle Dabringhausener Strasse 42, D-5093 Burscheid (DE)**
Erfinder: **Frohberger, Paul-Ernst, Dr., Willi-Baumeister-Strasse 5, D-5090 Leverkusen 1 (DE)**

(54) **Triazolyl-benzyloxy-ketone und -carbinole, Verfahren zu ihrer Herstellung sowie ihre Verwendung als Fungizide.**

(57) Die Erfindung betrifft neue Triazolyl-benzyl-oxyketone und -carbinole, mehrere Verfahren zu ihrer Herstellung sowie ihre Verwendung als Fungizide.

Die Verbindungen der Formel

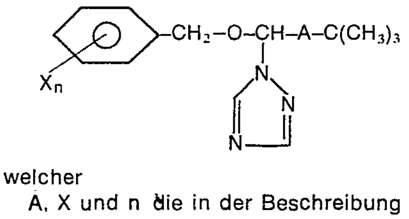

(I)

in welcher

A, X und n die in der Beschreibung angegebene Bedeutung besitzen, werden erhalten, wenn man Halogenetherketone mit 1,2,4-Triazol in Gegenwart eines Säurebindemittels und gegebenenfalls in Gegenwart eines Verdünnungsmittels umsetzt; außerdem gibt es noch andere Verfahrensweisen. Die erhaltenen Keto-Derivate können in üblicher Weise reduziert werden. Die Wirkstoffe sind für den Gebrauch als Pflanzenschutzmittel geeignet und können mit besonders gutem Erfolg zur Bekämpfung von Getreidekrankheiten, wie Getreidemehltau und Gerstenmehltau von Venturia-Arten, sowie zur Bekämpfung des echten Gurkenmehltaus eingesetzt werden.

EP 0 039 405 A2

0039405

BAYER AKTIENGESELLSCHAFT 5090 Leverkusen, Bayerwerk
Zentralbereich
Patente, Marken und Lizenzen Slr/Kü
I b

# Triazolyl-benzyloxy-ketone und -carbinole, Verfahren zu ihrer Herstellung sowie ihre Verwendung als Fungizide

Die vorliegende Erfindung betrifft neue Triazolyl-benzyloxy-ketone und -carbinole, mehrere Verfahren zu ihrer Herstellung sowie ihre Verwendung als Fungizide.

Es ist bereits bekannt geworden, daß im Phenylteil substituierte 3,3-Dimethyl-1-phenoxy-1-triazolyl-butan-2-one und -ole, wie beispielsweise 3,3-Dimethyl-1-(4-tert.-butylphenoxy)-1-(1,2,4-triazol-1-yl)-butan-2-on bzw. -ol und 3,3-Dimethyl-1-(pentachlorphenoxy)-1-(1,2,4-triazol-1-yl)-butan-2-on, im allgemeinen gute fungizide Eigenschaften aufweisen (vergleiche DE-AS 22 01 063 [LeA 14 118] und DE-OS 23 24 010 [LeA 14 971]). Die Wirkung ist jedoch, insbesondere bei niedrigen Aufwandmengen und -konzentrationen, in einigen Anwendungsbereichen nicht immer voll befriedigend.

Es wurden neue Triazolyl-benzyloxy-ketone und -carbinole der Formel

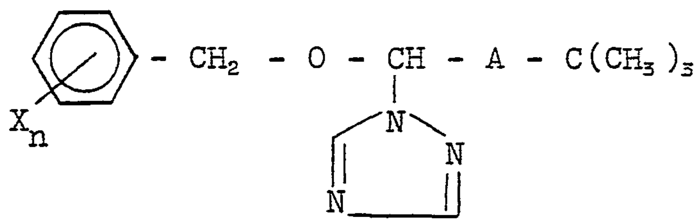 (I)

in welcher

A für die Ketogruppe oder eine CH(OH)-Gruppierung steht,

X für Halogen, Alkyl oder Halogenalkyl steht und

n für die Zahlen 0, 1, 2, 3, 4 oder 5 steht,

und deren physiologisch verträglichen Säureadditions-Salze sowie Metallsalz-Komplexe gefunden.

Diejenigen Verbindungen der Formel (I), in welchen A für die CH(OH)-Gruppe steht, besitzen zwei asymmetrische Kohlenstoffatomen; sie können deshalb in den beiden geometrischen Isomeren (erythro- und threo-Form) vorliegen, die in unterschiedlichen Mengenverhältnissen anfallen können. In beiden Fällen liegen sie als optische Isomeren vor. Sämtliche Iscmeren werden erfindungsgemäß beansprucht.

Weiterhin wurde gefunden, daß man die Triazolyl-benzyloxy-ketone und -carbinole der Formel (I) erhält, wenn man

a) Halogenetherketone der Formel

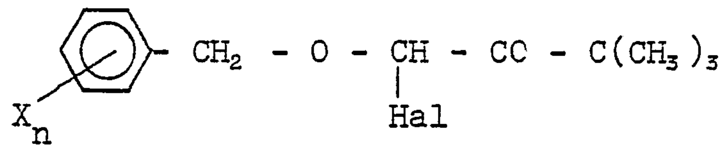 (II)

in welcher

X und n die oben angegebene Bedeutung haben und

Hal für Chlor oder Brom steht,

mit 1,2,4-Triazol in Gegenwart eines Säurebindemittels und gegebenenfalls in Gegenwart eines Verdünnungsmittels umsetzt, oder

b) Triazolylhalogenketone der Formel

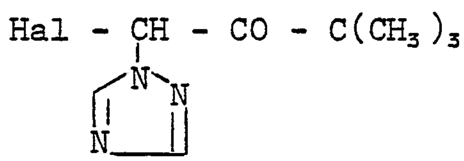 (III)

in welcher

Hal die oben angegebene Bedeutung hat,

mit Benzylalkoholen der Formel

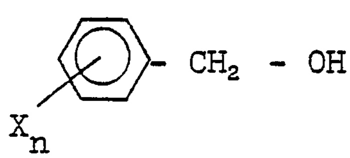 (IV)

in welcher

X und n die oben angegebene Bedeutung haben,

in Gegenwart eines Säurebindemittels und gegebenenfalls in Gegenwart eines Verdünnungsmittels umsetzt, und

c) gegebenenfalls die nach den Verfahrensvarianten (a) und (b) erhaltenen Keto-Derivate der Formel

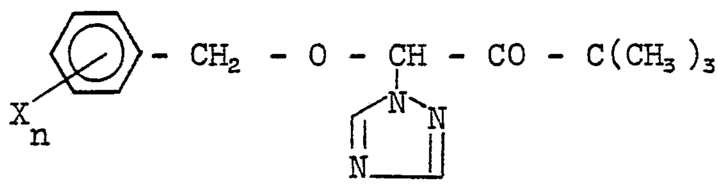

(Ia)

in welcher

X und n die oben angegebene Bedeutung haben,

nach bekannten Methoden in üblicher Weise reduziert.

An die so erhaltenen Verbindungen der Formel (I) kann gegebenenfalls anschließend eine Säure oder ein Metallsalz addiert werden.

Die neuen Triazolyl-benzyloxy-ketone und -carbinole der Formel (I) weisen starke fungizide Eigenschaften auf. Dabei zeigen überraschenderweise die erfindungsgemäßen Verbindungen eine bessere fungizide Wirksamkeit als die aus dem Stand der Technik bekannten Verbindungen 3,3-Dimethyl-1-(4-tert.-butyl-phenoxy)-1-(1,2,4-triazol-1-yl)-butan-2-on bzw. -ol und 3,3-Dimethyl-1-(pentachlorphenoxy)-1-(1,2,4-triazol-1-yl)-butan-2-on, welche chemisch und wirkungsmäßig naheliegende Verbindungen sind. Die erfindungsgemäßen Stoffe stellen somit eine Bereicherung der Technik dar.

Die erfindungsgemäßen Triazolyl-benzyloxy-ketone und -carbinole sind durch die Formel (I) allgemein definiert. In dieser Formel steht X vorzugsweise für Halogen, geradkettiges oder verzweigtes Alkyl mit 1 bis 4 Kohlenstoffatomen, sowie für Halogenalkyl mit 1 bis 2 Kohlenstoff- und 1 bis 5 gleichen oder verschiedenen Halogenatomen, wie insbesondere Fluor- und Chloratomen. Der Index n steht vorzugsweise für die Zahlen 0, 1, 2 und 3. A hat vorzugsweise die in der Erfindungsdefinition angegebene Bedeutung.

Ganz besonders bevorzugt sind diejenigen Verbindungen der Formel (I), in denen X für Fluor, Chlor, Brom, Methyl oder Trifluormethyl steht; der Index n für die Zahlen 0, 1 oder 2 steht; und A die in der Erfindungsdefinition angegebene Bedeutung hat.

Verwendet man beispielsweise 1-Brom-1-(4-chlorphenoxy)-3,3-dimethyl-butan-2-on und 1,2,4-Triazol als Ausgangsstoffe, so kann der Reaktionsablauf durch das folgende Formelschema wiedergegeben werden (Verfahren a):

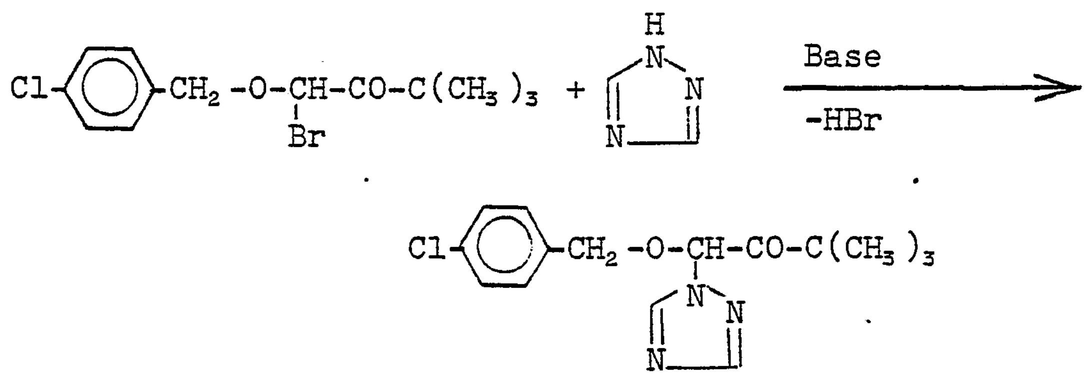

Verwendet man beispielsweise 1-Brom-3,3-dimethyl-1-(1,2,4-triazol-1-yl)-butan-2-on und 4-Chlorbenzylalkohol als Ausgangsstoffe, so kann der Reaktionsablauf durch das folgende Formelschema wiedergegeben werden (Verfahren b):

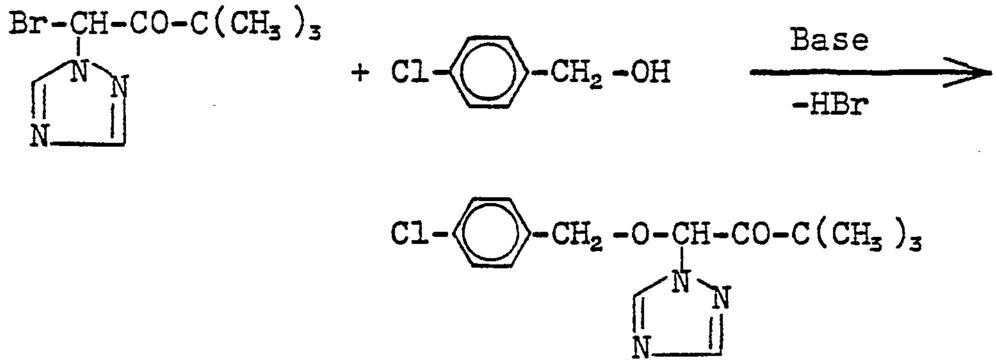


Verwendet man beispielsweise 1-(4-Chlorbenzyloxy)-3,3-dimethyl-1-(1,2,4-triazol-1-yl)-butan-2-on und Natriumborhydrid als Ausgangsstoffe, so kann der Reaktionsablauf durch das folgende Formelschema wiedergegeben werden (Verfahren c):

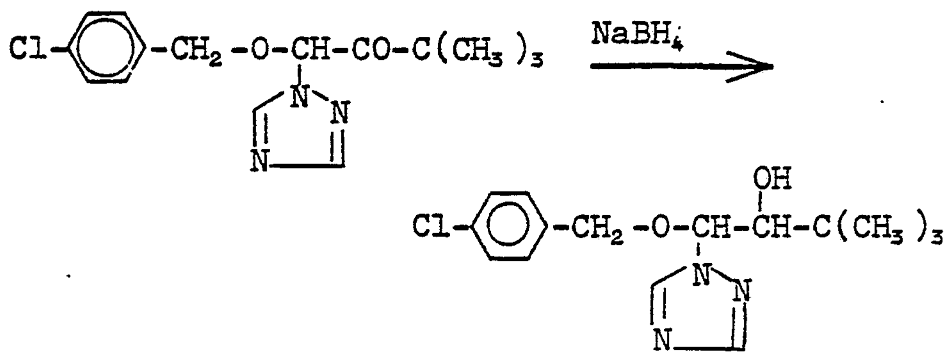


Die als Ausgangsstoffe für das Verfahren (a) zu verwendenden Halogenetherketone sind durch die Formel (II) allgemein definiert. In dieser Formel stehen X und der Index n vorzugsweise für die Reste, die bereits im Zusammenhang mit der Beschreibung der erfindungsgemäßen Stoffe der Formel (I) vorzugsweise für diese Substituenten genannt wurden.

Die Halogenetherketone der Formel (II) sind noch nicht bekannt; sie können aber nach bekannten Verfahren hergestellt werden, indem man z.B. bei Benzylethern der Formel

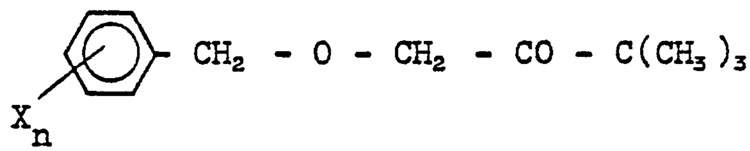 (V)

in welcher

X und n die oben angegebene Bedeutung haben,

eines der beiden aktiven Wasserstoffatome in üblicher Weise gegen Chlor oder Brom austauscht. Die entstehenden Halogenetherketone der Formel (II) können ohne Isolierung direkt weiter umgesetzt werden (vergleiche auch die Herstellungsbeispiele).

Die Benzylether der Formel (V) können nach bekannten Verfahren erhalten werden, indem man z.B. Benzylalkohole der Formel (IV) mit Chlor-(Brom)pinakolin der Formel

$$(Br)Cl - CH_2 - CO - C(CH_3)_3 \qquad (VI)$$

in Gegenwart einer starken Base, wie z.B. Natriumhydrid, und in Gegenwart eines inerten organischen Lösungsmittels, wie z.B. Dimethylformamid, bei Temperaturen zwischen 20 und 100°C umsetzt; oder indem man Benzylhalogenide der Formel

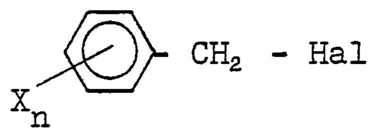 (VII)

in welcher

Hal, X und n die oben angegebene Bedeutung haben,

mit Hydroxypinakolin der Formel

$$HO - CH_2 - CO - C(CH_3)_3$$

in einem Zweiphasensystem, wie z.B.wässrige Natron- oder Kalilauge/Toluol oder Methylenchlorid, unter Zusatz eines Phasentransfer-Katalysators, wie beispielsweise Ammoniumverbindungen wie Triethyl-benzyl-ammoniumchlorid, umsetzt.

Die als Ausgangsstoffe für das Verfahren(b) zu verwendenden Triazolylhalogenketone sind durch die Formel (III) allgemein definiert und bekannt (vergleiche DE-OS 27 56 269 [LeA 18 565]). Sie werden erhalten, indem man Chlor-(Brom)pinakolin der Formel (VI) mit 1,2,4-Triazol in Gegenwart eines Säurebindemittels, wie z.B. Kaliumcarbonat, und in Gegenwart eines inerten organischen Lösungsmittels, wie z.B. Aceton, bei Temperaturen zwischen 60 und 120°C umsetzt. Eines der beiden aktiven Wasserstoffatome wird anschließend in üblicher Weise gegen Chlor oder Brom ausgetauscht.

Die außerdem für das Verfahren (b) als Ausgangstoffe zu verwendenden Benzylalkohole sind durch die Formel (IV) allgemein definiert. In dieser Formel stehen X und der Index n vorzugsweise für die Reste, die bereits im Zusammenhang mit der Beschreibung der erfindungsgemäßen Stoffe der Formel (I) vorzugsweise für diese Substituenten genannt wurden.

Die Benzylalkohole der Formel (IV) sind allgemein bekannte Verbindungen der organischen Chemie.

Für die erfindungsgemäße Umsetzung gemäß Verfahrensvarianten (a) und (b) kommen als Verdünnungsmittel inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Terahydrofuran oder Dioxan; Benzol; Formamide; wie insbesondere Dimethylformamid; und halogenierte Kohlenwasserstoffe.

Die Umsetzungen nach Verfahren (a) und (b) werden in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat oder wie Silbercarbonat, oder wie niedere tertiäre Alkylamine, Cycloalkylamine oder Aralkylamine, beispielsweise Triethylamin, Dimethylbenzylamin; oder wie Pyridin und Diazabicyclooctan.
Bei der Verfahrensvariante (a) kann auch ein entsprechender Ueberschuß an Azol verwendet werden.

Die Reaktionstemperaturen können bei den Verfahren (a) und (b) in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung der erfindungsgemäßen Verfahren (a) bzw. (b) setzt man auf 1 Mol der Verbindung der Formel (II) bzw. (III) vorzugsweise 1 bis 2 Mol 1,2,4-Triazol

bzw. 1 bis 2 Mol Benzylalkohol der Formel (IV) und jeweils 1 bis 2 Mol Säurebinder ein. Zur Isolierung der Verbindungen der Formel (I) wird das Lösungsmittel abdestilliert und der Rückstand entweder mit Wasser versetzt und kräftig gerührt, wobei das Reaktionsprodukt durchkristallisiert, oder mit einem Gemisch aus einem organichen Solvens und Wasser aufgenommen, die organische Phase abgetrennt, mit Wasser gewaschen, über Natriumsulfat getrocknet und im Vakuum vom Lösungsmittel befreit. Der Rückstand wird gegebenenfalls durch Destillation oder Umkristallisation gereinigt.

Die erfindungsgemäße Reduktion gemäß Verfahrensvariante (c) erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketones der Formel (Ia) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminium-Verbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren,wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B.durch Lösen des Metallsalzes in Alkohol,z.B.Ethanol und Hinzufügen zur Verbindung der Formel (I).Man kann Metallsalz-Komplexe in bekannter Weise,z.B. durch Abfiltrieren,Isolieren und gegebenenfalls durch Umkristallisation reinigen.

0039405

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Getreidemehltau und Gerstenmehltau; von Venturia-Arten, wie gegen den Erreger des Apfelschorfs (Fusicladium dendriticum); sowie zur Bekämpfung des echten Gurkenmehltaus (Erysiphe cichoriacearum) eingesetzt werden. Besonders hervorzuheben ist, daß die erfindungsgemäßen Wirkstoffe nicht nur eine protektive Wirkung haben, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

0039405

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.
In entsprechenden Aufwandmengen zeigen die erfindungsgemäßen Verbindungen auch eine wachstumsregulierende Wirkung.

Herstellungsbeispiele

Beispiel 1

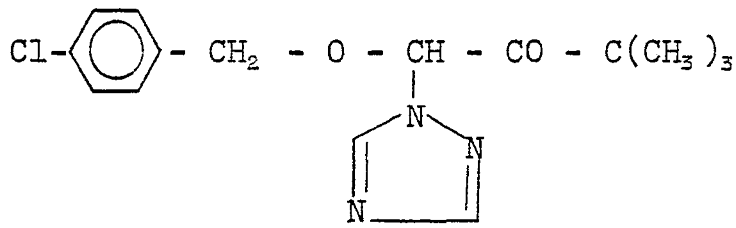

(Verfahren a)

31,9g (0,1 Mol) rohes 1-Brom-1-(4-chlorbenzyl)-3,3-dimethyl-butan-2-on in 100 ml Acetonitril werden bei Raumtemperatur unter Rühren mit 20,7g (0,3 Mol) 1,2,4-Triazol versetzt. Man läßt 1 Stunde unter Rückfluß rühren, engt durch Abdestillieren des Lösungsmittels ein und nimmt den Rückstand mit Methylenchlorid/Wasser auf. Die organische Phase wird abgetrennt, nochmals mit Wasser gewaschen, über Natriumsulfat getrocknet und eingeengt. Nach einer säulenchromatographischen Reinigung erhält man 12,4g (45 % der Theorie) 1-(4-Chlorbenzyloxy)-3,3-dimethyl-1-(1,2,4-triazol-1-yl)-butan-2-on als viskoses Oel.

Herstellung des Vorproduktes

(II-1) 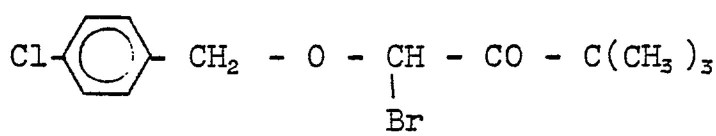

24g (0,1 Mol) 1-(4-Chlorbenzyloxy)-3,3-dimethyl-butan-2-on in 100ml Methylenchlorid werden bei Raumtemperatur so mit 16g (0,1 Mol) Brom versetzt, daß stetige Entfärbung eintritt. Anschließend wird das Reaktionsgemisch mit Wasser und verdünnter Natriumhydrogencarbonatlösung gewaschen, über Natriumsulfat getrocknet und bei 40°C im Vakuum durch Abdestillieren des Lösungsmittels eingeengt. Man erhält quantitativ rohes 1-Brom-1-(4-chlorbenzyloxy)-3,3-dimethyl-butan-2-on, das direkt weiter umgesetzt wird.

(V-1) 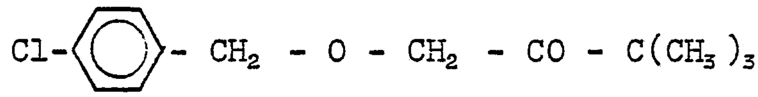

88,8g (0,6 Mol) 4-Chlorbenzylchlorid und 24 g (0,2 Mol) Hydroxypinakolin werden in 150 ml Toluol gelöst und nach Zugabe von 10 ml Triethyl-benzyl-ammoniumchlorid sowie 100 ml 30%-iger Natronlauge 12 Stunden heftig gerührt. Danach wird die organische Phase abgetrennt, gewaschen, über Natriumsulfat getrocknet und destilliert. Man erhält 33,6g (70 % der Theorie) 1-(4-Chlorbenzyloxy)-3,3-dimethyl-butan-2-on vom Siedepunkt 125°C/0,1 Torr.

Beispiel 2

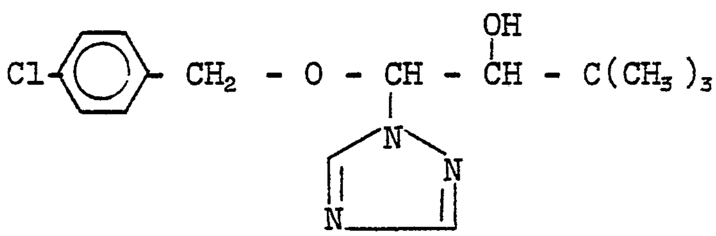

(Verfahren c)

Zu 30,7g (0,1 Mol) 1-(4-Chlorbenzyloxy)-3,3-dimethyl-1-(1,2,4-triazol-1-yl)-butan-2-on (Beispiel 1) in 200 ml Ethanol gibt man bei Raumtemperatur unter Rühren 3,8g (0,1 Mol) Natriumborhydrid. Sobald die exotherme Reaktion abgeklungen ist, erhitzt man das Reaktionsgemisch 1 Stunde auf 60°C. Danach wird eingeengt und der Rückstand zwischen Methylenchlorid/Wasser verteilt. Die organische Phase wird abgetrennt, mit Wasser gewaschen, über Natriumsulfat getrocknet und eingeengt. Der Rückstand wird nach säulenchromatographischer Vorreinigung aus Cyclohexan umkristallisiert. Man erhält 15,4g (50 % der Theorie) 1-(4-Chlor-benzyl)-3,3-dimethyl-1-(1,2,4-triazol-1-yl)-butan-2-ol vom Schmelzpunkt 96°C.

Analog und gemäß den Verfahrensvarianten (a), (b) und (c) werden die folgenden Verbindungen der allgemeinen Formel

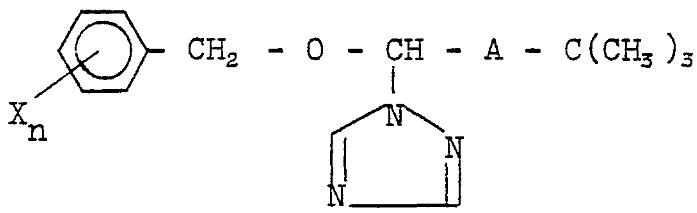

(I)

erhalten:

| Bsp.Nr. | $X_n$ | A | Schmelzpunkt(°C) |
|---|---|---|---|
| 3 | - | CO | 43-48 |
| 4 | 3,4-$Cl_2$ | CO | 97 |
| 5 | 2,6-$Cl_2$ | CO | 119 |
| 6 | - | CH(OH) | 74 |
| 7 | 3,4-$Cl_2$ | CH(OH) | 55-61 |
| 8 | 2,6-$Cl_2$ | CH(OH) | 125 |

Entsprechend Beispiel 1 und gemäß der allgemeinen Beschreibung werden die folgenden Zwischenprodukte der allgemeinen Formel

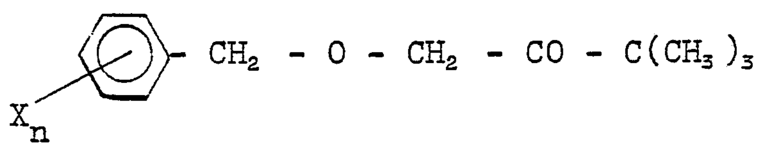

(V)

erhalten:

| Bsp.Nr. | $X_n$ | Siedepunkt(°C)/mm Hg-Säule |
|---|---|---|
| (V-2) | - | 110/0,9 |
| (V-3) | 3,4-$Cl_2$ | 140/0,1 |
| (V-4) | 2,6-$Cl_2$ | 120/0,1 |

Anwendungsbeispiele

In den nachfolgenden Beispielen werden die nachstehend angegebenen Verbindungen als Vergleichssubstanzen eingesetzt.

(A) = 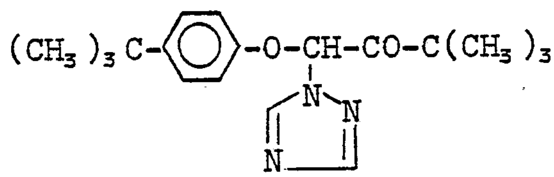

(B) = 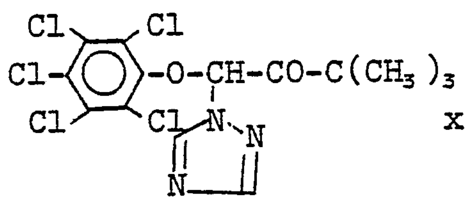 x $H_2O$

(C) = 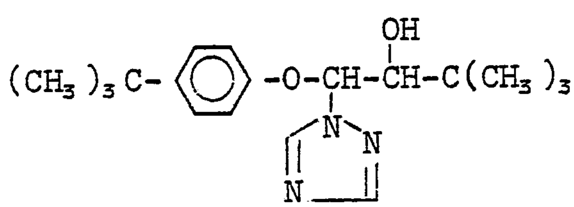

Beispiel A

Sproßbehandlungs-Test/ Getreidemehltau/ Protektiv
(blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.
Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.
Nach 6 Tagen Verweilzeit der Pflanzen bei einer Temperatur von 21-22°C und einer Luftfeuchtigkeit von 80-90% wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.
Bei diesem Test zeigen z.B. die folgenden Verbindungen eine sehr gute Wirkung, die derjenigen der aus dem Stand der Technik bekannten Verbindungen(A) und (B) überlegen ist:
Verbindungen gemäß Herstellungsbeispielen 6,2,7,3,4, 1 und 5.

## T a b e l l e A

Sproßbehandlungs-Test/ Getreidemehltau/ Protektiv

| W i r k s t o f f e | Wirkstoffkonzentration in der Spritzbrühe in Gew.% | Befall in % der unbehandelten kontrolle |
|---|---|---|
| 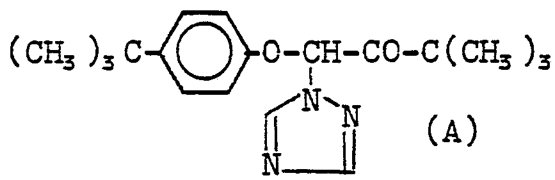 (A)<br>(bekannt) | 0,01 | 82,5 |
| 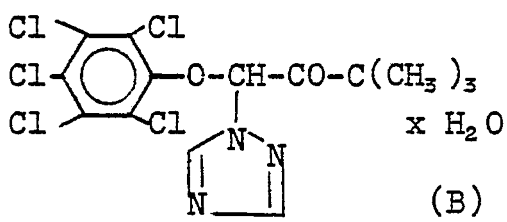 (B)<br>(bekannt) | 0,01 | 91,3 |
| 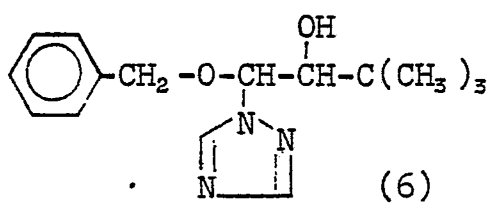 (6) | 0,01 | 15,0 |
| 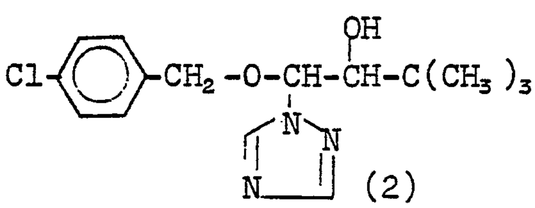 (2) | 0,025<br>C,0025 | 0,0<br>15,0 |
| 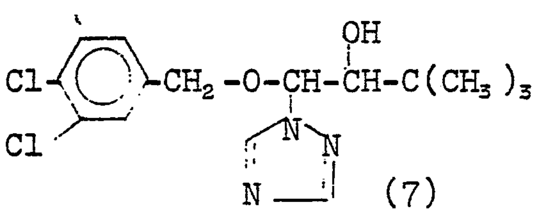 (7) | 0,025<br>0,0025 | 0,0<br>12,5 |

T a b e l l e A(Fortsetzung)

Sproßbehandlungs-Test/ Getreidemehltau/ Protektiv

| W i r k s t o f f e | Wirkstoffkonzentration in der Spritzbrühe in Gew.% | Befall in % der unbehandelten kontrolle |
|---|---|---|
| 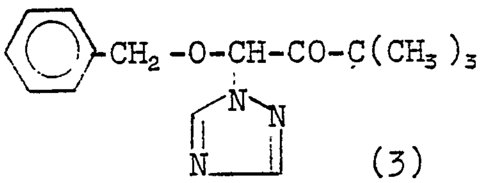 (3) | 0,01 | 48,8 |
| 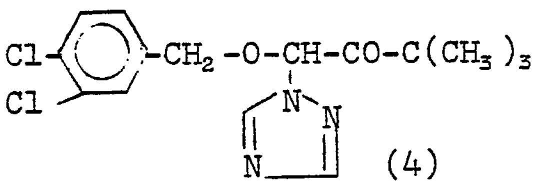 (4) | 0,01 | 0,0 |
| 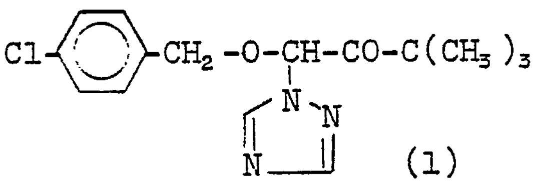 (1) | 0,01 | 5,0 |
| 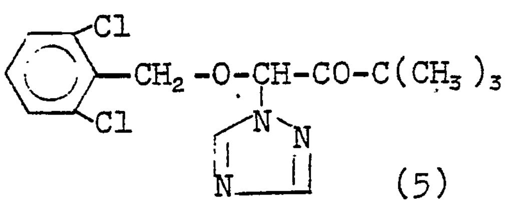 (5) | 0,01 | 15,0 |

Beispiel B

Gerstenmehltau-Test (Erysiphe graminis var.hordei)/ systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var.hordei bestäubt und bei 21-22°C und 80-90% rel.Luftfeuchte und 16-stündiger Belichtung weiter kultiviert. Innerhalb von 6 Tagen bilden sich an den Blättern die typischen Mehltaupusteln aus.
Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Bei diesem Test zeigen z.B. die folgenden Verbindungen eine sehr gute Wirkung, die derjenigen der aus dem Stand der Technik bekannten Verbindungen (A) und (C) überlegen ist:
Verbindungen gemäß Herstellungsbeispielen: 6,2,7,3 und 4.

Tabelle B

Gerstenmehltau-Test (Erysiphe graminis var.hordei)/
systemisch

| Wirkstoffe | Wirkstoffkonzentration im Beizmittel in Gew.% | Beizmittelaufwandmenge in g/kg Saatgut | Befall in % der unbehandelten Kontrolle |
|---|---|---|---|
| 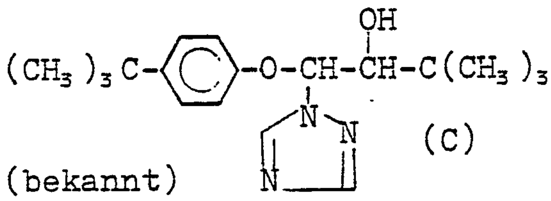 (C) (bekannt) | 25 | 10 | 100 |
| 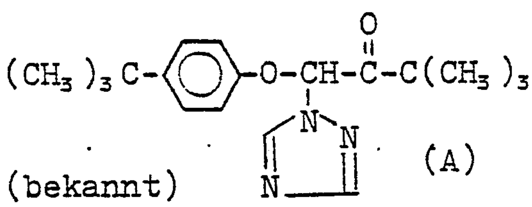 (A) (bekannt) | 25 | 10 | 100 |
| 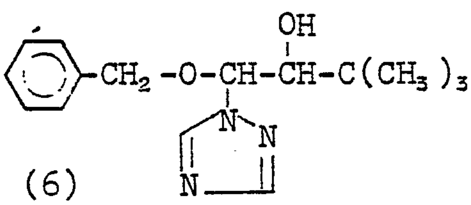 (6) | 25 | 10 | 0,0 |
| 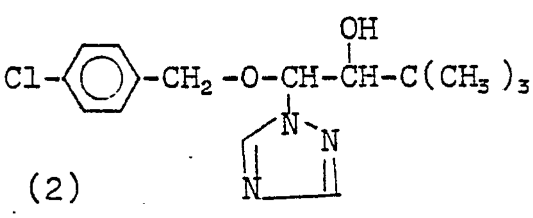 (2) | 25 | 10 | 3,8 |
| 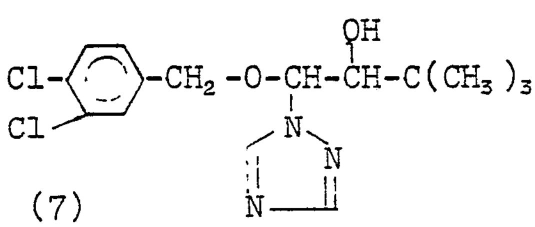 (7) | 25 | 10 | 0,0 |

Tabelle B (Fortsetzung)

Gerstenmehltau-Test (Erysiphe graminis var.hordei)/ systemisch

| Wirkstoffe | Wirkstoffkonzentration im Beizmittel in Gew.% | Beizmittelaufwandmenge in g/kg Saatgut | Befall in % der unbehandelten Kontrolle |
|---|---|---|---|
| 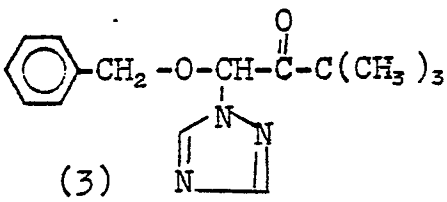 (3) | 25 | 10 | 12,5 |
| 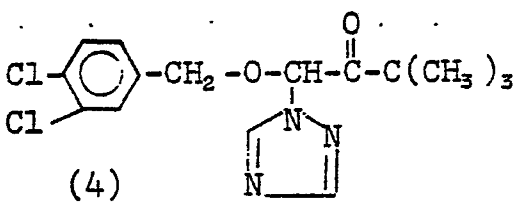 (4) | 25 | 10 | 0,0 |

Patentansprüche

1) Triazolyl-benzyloxy-ketone und -carbinole der Formel

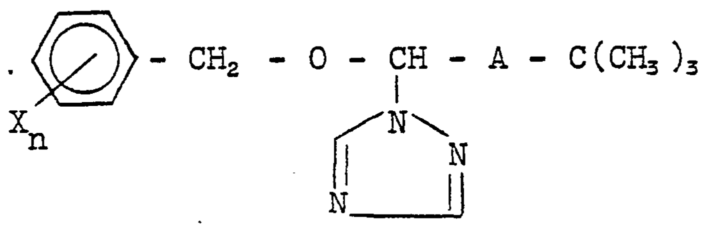

(I)

in welcher

A für die Ketogruppe oder einen CH(OH)-Gruppierung steht,

X für Halogen, Alkyl oder Halogenalkyl steht und

n für die Zahlen 0, 1, 2, 3, 4 oder 5 steht,

und deren physiologisch verträglichen Säureadditions-Salz sowie Metallsalz-Komplexe.

2) Verfahren zur Herstellung von Triazolyl-benzyloxy-ketonen und -carbinolen der Formel I, dadurch gekennzeichnet, daß man

a) Halogenetherketone der Formel

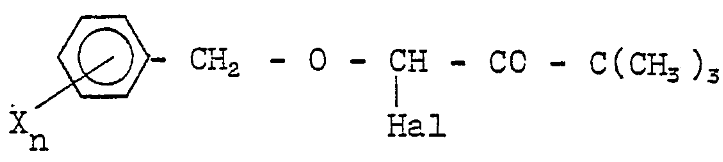

(II)

in welcher

X und n die in Anspruch 1 angegebene Bedeutung haben und

Hal für Chlor oder brom steht,

mit 1,2,4-Triazol in Gegenwart eines Säurebindemittels und gegebenenfalls in Gegenwart eines Verdünnungsmittels umsetzt, oder

b) Triazolylhalogenketone der Formel

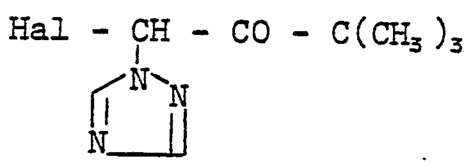

(III)

in welcher

Hal die in Anspruch 1 angegebene Bedeutung hat,

mit Benzylalkoholen der Formel

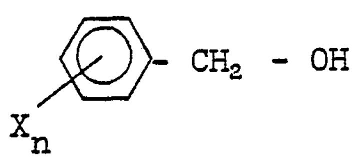

(IV)

in welcher

X und n die in Anspruch 1 angegebene Bedeudeutung haben,

in Gegenwart eines Säurebindemittels und gegebenenfalls in Gegenwart eines Verdünnungsmittels umsetzt, und

c) gegebenenfalls die nach den Verfahrensvarianten (a) und (b) erhaltenen Keto-Derivate der Formel

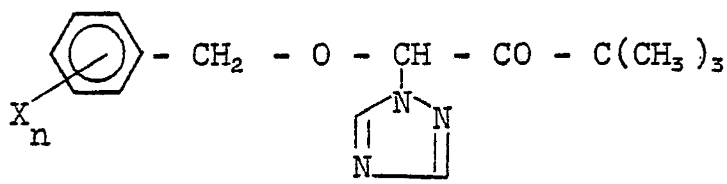 (Ia)

in welcher

X und n die oben angegebene Bedeutung haben,

nach bekannten Methoden in üblicher Weise reduziert,

und gegebenenfalls noch an die so erhaltenen Verbindungen der Formel (I) anschließend eine Säure oder ein Metallsalz addiert.

3) Fungizide Mittel, gekennzeichnet durch einen Gehalt an mindestens einem Triazolyl-benzyloxy-keton oder -carbinol der Formel I.

4) Verfahren zur Bekämpfung von Pilzen dadurch gekennzeichnet, daß Triazolyl-benzyloxy-ketone oder -carbinole der Formel I auf Pilze oder ihren Lebensraum einwirken läßt.

5) Verwendung von Triyzolyl-benzyloxy-ketonen oder -carbinolen der Formel I zur Bekämpfung von Pilzen.

6) Verfahren zur Herstellung von fungiziden Mitteln, dadurch gekennzeichnet, daß man Trizolylbenzyloxyketone oder -carbinole der Formel I mit Streckmitteln und/oder oberflächenaktiven Mitteln vermischt.